# EUROPEAN PATENT SPECIFICATION

(11) **EP 0 374 157 B1**
(45) Date of publication and mention of the grant of the patent: **06.07.1994**
(21) Application number: 88905520.8
(22) Date of filing: 31.05.1988
(51) Int. Cl.: C07K 1/06, C07K 3/04, C07K 7/10, C07K 13/00, A23L 1/236, C07H 21/04, C12N 1/20, C12P 21/02

(54) **A NEW CLASS OF LOW CALORIE PROTEIN SWEETENERS**
NEUE KLASSE VON NIEDRIGKALORISCHEN PROTEINSÜSSSTOFFEN
NOUVELLE CLASSE D'EDULCORANTS A BASE DE PROTEINE A FAIBLE TAUX DE CALORIES

(30) Priority: 19.06.1987 US 64341; 19.06.1987 US 64343; 04.11.1987 US 117124
(43) Date of publication of application: 27.06.1990
(73) Proprietor: THE REGENTS OF THE UNIVERSITY OF CALIFORNIA, Oakland, California 94612-3550 (US); LUCKY, LTD., Seoul 150-010 (KR)
(72) Inventor: KIM, Sung-Hou, Moraga, CA 94556 (US); Cho, Joong Myung, Concord, California 94521 (US)
(74) Representative: Perry, Robert Edward
(86) International application number: US8801825
(87) International publication number: WO8810265

(56) References cited:
- WO-A-88/10271
- US-A- 3 878 184
- US-A- 4 332 892
- US-A- 4 440 859
- B.J.F. HUDSON: "Developments in food proteins-4", Elsevier Applied Science Publishers, London, GB; Van der Wel: "The sweet protein", (Chapter 6, pages 219-245)
- Lehninger,A.L., Biochemistry,1st ed. (1970) Worth publishers Inc. (New York. N.Y., U.S.A.) ,pages 103 and 104.
- Biochemical and Biophysical Research Communications, Vol.71, no. 1, 1976,(Duluth, Minnesota, U.S.A),G Hudson et al., "Mass Spectrometric Sequencing of Proteins. The Structure of Subunit I of Monellin", pages 212-220(note figure 1).
- Biochemica et Biophysica Acta, Vol. 427, 1976, (Duluth, Minnesota, U.S.A.) Z Bohak et al., "The Structure of Monellin and its Relation to the Sweetness of the Protein", page 153-170 (note pages 168 and 169).
- Federation Proceedings, Vol. 33, no. 10, October 1974, (Bethesda Maryland, U.S.A.), D.F. Steiner et al., "Proteolytic Processing in the Biosynthesis of Insulin and Other Proteins", pages 2105-2115(Note page 2108 and 2109)
- Nature, Vol.271, 26 January 1978 (Tokyo, Japan),C.A.M. Hough et al., "Antibodies to Thaumatin as a model of the sweet taste receptor", pages 381-383 (Note page 382).
- Chemical Abstracts, Vol. 85, no. 3, issued 1976, July 19 (Columbus, Ohio, USA), G. Frank et al., "The complete amino acid sequences of both subunits of the Sweet Protein Monnellin", see page 210, colume 1, the abstract no. 15663x,(Hoppe-Seyler's Z. Physiol. Chem.1976,357(4), 585-92(Eng)).
- Chemical Abstracts, Vol. 108, No. 7 issued 1988, February 15 (Columbus, Ohio, U.S.A. ), Y.P. Vinetskii, "Sweet Proteins and their Genes", see page 273, colume 2, the abstract no. 51330p, (Biotekhnologiya, 1987 3(5), 553-8 (Russ)).

## Description

### Technical Field

The invention relates to using proteins as substitutes for sugar in sweetening foods and beverages. In particular, it concerns single chain proteinaceous compounds which are much sweeter than sucrose, and which retain their sweetening capacity although subjected to denaturing conditions.

### Background of the Invention

It is well known that certain proteinaceous compounds have the ability to substitute in a highly effective manner for sugar in giving foods and beverages a sweet taste. The simplest of these examples is aspartame, which is a dipeptide derivative and currently on the market. However, two much more complex proteins, monellin and thaumatin have been isolated from plant sources. Thaumatin is isolated from Thaumatococcus daniellii, a West African plant having triangular shaped fruit at ground level. The natural protein product, thaumatin, has an average sweetness of 2500 times that of sucrose and has been marketed under the trademark Talin. The three dimensional structure of this protein has been studied and the results published by De Vos, A.M., et al, Proc Natl Acad Sci USA (1985) 82:1406-1409.

The other protein, which is nonglycosylated, is isolated from "Serendipity Berries" of the West African Plant Dioscoreophyhllum comminisii. The amino acid sequence of monellin is known, and the three dimensional structure of this protein has been determined by Ogata, C., et al, Nature (1987) 328:739-742. Monellin has been characterized by Morris et al, J Biol Chem (1973) 248:534-539, and by others; Cagan, Science (1973) 181:32-35; Bohak and Li, Biochim Biophys Acta (1976) 427:153-170; Hudson and Beeman, Biochem Biophys Res Comm (1976) 71:212-220; Van der Wel and Loeve, FEBS Lett (1973) 29:181-183; Frank and Zuber Hoppe-Seyler's Z Physiol Chem (1976) 357:585-592; Morris and Cagan, Biochim Biophys Acta (1972) 261:114-122. U.S. Patent 3,998,798 describes the preparation of natural monellin.

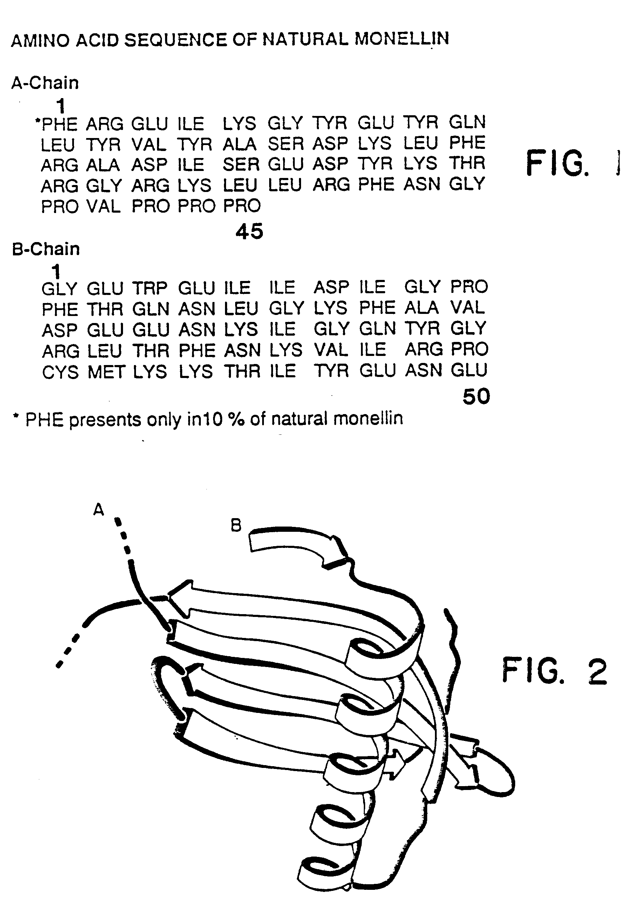

The known amino acid sequence of the A and B chains of natural monellin is shown in Figure 1. It is a two chain peptide, one "A" chain containing 45, and the other "B" chain, 50 amino acid residues. The three dimensional conformation of the protein, shown in Figure 2, is evidently essential for its activity because when native monellin is heated to 90°C at neutral pH or to 50°C at acidic pH and then cooled, the sweetness is destroyed.

The three-dimensional conformation of the protein as recently determined (supra) is shown in Figure 2. A B chain containing 50 amino acids is intimately associated with the A chain of 45 amino acids in such a way that there are many inter-chain interactions. Heating of the protein, evidently dissociates the chains in such a way that they are incapable of reforming into the appropriate conformation.

It has now been found that the conformation of this proteinaceous compound can be maintained by synthesizing portions of the B and A components on a single molecule. This constraint results in resistance to denaturation and ease of maintaining the three dimensional conformation.

### Disclosure of the Invention

The invention provides single chair forms of the sweet proteinaceous substance, monellin, which are capable of maintaining their three dimensional conformation under conditions which would ordinarily denature the native protein. These forms retain their sweet taste, which is substantially more intense than that of sucrose, even after heating to 100°C at acidic pH. Thus, the substance remains useful for sweetening of foods and beverages including carbonated beverages exposed to temperatures above ambient.

In one aspect the invention is directed to a single chain proteinaceous compound having a sweetness property at least 50, preferably 100, and most preferably 1000 times that of sucrose on a weight basis. The compound has the formula B-C-A wherein B is substantially equivalent to the residues 1-46 of the B chain portion of native monellin and is linked through the C-terminus to C. C is a covalent linker which is, in turn, linked to the N-terminus of a peptide substantially equivalent to residues 6-45 of the A chain of native monellin. The covalent linker, C, must be of approximately the length of 3-10 alpha-amino acid peptide, of sufficient hydrophility to reside on the outer side of the molecules, and physiologically acceptable. If the covalent linker is itself a peptide the substance will be a single chain protein which can be prepared using automated synthetic methods or can be prepared from a synthetic gene using recombinant techniques, or by other methods known in the art.

As recombinant methods may also be used, the invention, in another aspect, includes DNA sequences encoding the protein, expression vectors containing these sequences, microorganisms or other host cells transformed with these vectors, and methods to produce the protein using these materials. The invention also includes useful intermediates in the synthesis of the compounds of the invention.

### Brief Description of the Drawings

Figure 1 shows the amino acid sequence of the A and B chains of the native protein.

Figure 2 is a representation of the three dimensional conformation of the native protein.

Figure 3 shows the amino acid sequence of a single chain protein of the invention, and the nucleotide sequence of a synthetic gene useful in synthesizing said single chain protein.

### Modes of Carrying Out the Invention

The proteinaceous compounds of the invention, useful as sweeteners, consist essentially of a peptide portion substantially equivalent to the sequence of residues 1-45 of the B chain of native monellin linked through the C-terminus by a covalent linker to the N-terminus of a peptide substantially equivalent to the sequence of residues 6-45 of A chain of native monellin. By "substantially equivalent" is meant a peptide which, in the context of the compounds of the invention, results in a substance having a sweetening power at least 50 times that of sucrose and which has at least 80% homology with the peptide represented by residues 1-46 of the B chain or to the peptide represented by residues 6-45 of the A chain. At least 90% homology is preferred especially including conservative substitutions.

Homology is calculated by standard methods which involve aligning two sequences to be compared so that maximum matching occurs, and calculating the percentage of matches. Thus, in a particularly preferred embodiment, the substances of the invention comprise a peptide having the amino acid sequence of residues 1-46 of the native monellin B chain linked (through the linker) to a peptide having the primary structure represented by residues 6-45 of the native monellin A chain. Substantially equivalent substances to these include those wherein one or more of the residues of the native sequence is deleted, substituted for, or inserted by a different amino acid or acids.

Preferred substitutions are those which are conservative, i.e., wherein a residue is replaced by another of the same general type. As is well understood, naturally occurring amino acids can be subclassified as acidic, basic, neutral and polar, or neutral and nonpolar. Furthermore, three of the encoded amino acids are aromatic. It is generally preferred that peptides differing from the native sequence contain substitutions which are from the same group as that of the amino acid replaced. Thus, in general, the basic amino acids Lys, Arg, and His are interchangeable; the acidic amino acids aspartic and glutamic are interchangeable; the neutral polar amino acids Ser, Thr, Cys, Gln, and Asn are interchangeable; the nonpolar aliphatic acids Gly, Ala, Val, Ile, and Leu are interchangeable; and the aromatic amino acids Phe, Trp, and Thr are interchangeable. While proline is a nonpolar neutral amino acid, it represents difficulties because of its effects on conformation, and substitutions by or for proline are not preferred, except when the same or similar conformational results can be obtained. In addition, although classified in different categories, Ala, Gly, and Ser seem to be interchangeable, and Cys additionally fits into this group. Some substitutions by amino acids from different classes may be useful to modify sweet taste responses.

It should further be noted that substitutions by amino acids which are not encoded by the gene may also be made. Alternative residues include, for example, the omega amino acids of the formula H₂N(CH₂)ₙCOOH wherein n is 2-6. These are neutral, nonpolar amino acids, as are sarcosine (Sar), t-butyl alanine (t-BuA), t-butyl glycine (t-BuG), N-methyl Ile (N-MeIle), and norleucine (Nle). Phenyl glycine, for example, can be substituted for Trp, Tyr or Phe an aromatic neutral amino acid; citrulline (Cit) and methionine sulfoxide (MSO) are polar but neutral, cyclohexyl alanine (Cha) is neutral and nonpolar, cysteic acid (Cya) is acidic, and ornithine (Orn) is basic. The conformation conferring properties of the proline residues may be retained if one or more of these is substituted by hydroxyproline (Hyp).

In addition, one or more of the peptide linkages in the B or A peptide-corresponding portions of the substances of the invention may be replaced with other types of linkages which retain the same general properties such as -CH₂NH-, -CH₂S-, -CH₂CH₂-, -CH=CH-, -COCH₂-, -CH(OH)CH₂-, and -CH₂SO- by methods known in the art. See, for example, Spatola, A.F. Vega Data (1983), 1(3), "Peptide Backbone Modifications"; Spatola, A.F., in "Chemistry and Biochemistry of Amino Acids, Peptides and Proteins", B. Weinstein, ed., Marcel Dekker, New York, p 273 (1983); Morley, J.S., Trans Pharm Sci (1980), pp 463-468. These substitutions may be made so long as the resulting "protein" is physiologically acceptable.

It should be further noted that if the protein embodiments of the invention are produced recombinantly as intracellular proteins, and N-terminal methionine residue may be retained in the finished product. Cleavage of this N-terminal methionine to liberate the native sequence may or may not be complete.

The covalent linkers of the invention are, generally, of a length equivalent to that generated by a peptide of 3-10 amino acid residues, preferably 6-8 amino acid residues, and must be physiologically acceptable. By "physiologically acceptable" is meant non-toxic and free of undesirable side effects. The covalent linker should have a hydrophilicity and length which permits the covalent linker to reside on the external portion of the molecule and not to distort the native conformation. One preferred covalent peptide linking sequence is shown below:
Tyr-Glu-Asn-Glu-Arg-Glu-Ile-Lys.

A particularly preferred group of covalent linkers represented by C comprises a peptide sequence containing 3-10, preferably 6-8, of the formula
A1-A2-A3-A4-A5-A6-A7-A8-A9-A10
wherein each A1-A10 may be an amino acid residue or may be absent, but at least three of A1-A10 must be amino acid residues. In a particularly preferred group of embodiments, A9 and A10 are absent, A2, A4 and A6 are acidic amino acids; A5 and A8 are basic amino acids, A3 is a polar/neutral amino acid, and A1 and A7 are nonpolar amino acids. In a particularly preferred set of embodiments, A9 and A10 are absent, A1 is Tyr or Phe, A2 is Glu or Asp, A3 is Asn or Gln, A4 is Glu or Asp, A5 is Arg, His, or Lys, A6 is Glu or Asp, A7 is Ile, Val, or Leu, and A8 is Arg, Lys, or His.

Unless otherwise noted, the amino acid residues whether encoded by the gene or as nonencoded analogs, are in the L-configuration. However, two of such residues, preferably one, may be substituted by the D-isomer.

Alternative linkers include any covalent linking moiety which is of the appropriate length as described above, appropriate hydrophilicity, and physiological acceptability. For example, polyethylene glycol oligomers of the formula HO(CX₂CX₂O)ₙH wherein each X is independently H or a saturated or unsaturated hydrocarbyl of 1-4C and wherein n is 3-10, preferably 6-8, can also be used. Oxidation of one of the terminal hydroxyl groups provides a carboxyl for formation of the amide to the N-terminus of the A chain; linkage to the B chain is through an ester linkage with the remaining hydroxyl of the polyethylene glycol. A variety of covalent linkers of the correct functionality, hydrophilicity and length can be provided and used to effect covalent linkage of the C-terminus of the B chain with the N-terminus of the A chain. A variety of covalent linkers, for example, is available commercially from Pierce Chemical Company.

The B and A peptides of the invention compounds, and the C linking portion, if it, too, is a peptide, can be synthesized using standard automated peptide synthesis techniques, either manually or on automated synthesizers such as those supplied by Applied Biosystems (Foster City, California), or Biosearch (San Rafael, California). Resins derivatized to the C-terminal amino acid in protected form are commercially available. Thus, for synthesis of the A chain portion, resins derivatized with proline are preferred; for the B portion, in a preferred embodiment, those derivatized to Ile. The subsequent amino acids are added using standard techniques to synthesize peptides of the required length. The A and B chains can each be synthesized in toto or as fragments which are then ligated, and subsequently linked with the covalent linker to obtain the substances of the invention.

If prepared using recombinant techniques, a DNA sequence encoding the desired peptide portion, in some cases the full-length peptide, is synthesized using standard automated techniques. This DNA is ligated into suitable expression vectors and these vectors are transformed into appropriate hosts. A variety of expression vector/host cell systems can be used, including both procaryotic and eucaryotic culture systems.

Procaryotes most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication origins, and control sequences derived from a species compatible with the host are used. For example, E. coli is typically transformed using derivatives of pBR322, a plasmid derived from an E. coli species by Bolivar et al, Gene (1977) 2:95. Commonly used procaryotic control sequences, which are defined herein to include promoters for transcription initiation, optionally with an operator, along with ribosomo binding site sequences, include such commonly used promoters as the beta-lactamase (penicillinase), lactose (lac) promoter systems (Chang et al, Nature (1977) 198:1056), the tryptophan (trp) promoter system (Goeddel et al, Nucleic Acids Res (1980) 8:4057), and the lambdaderived P_{L} promoter and N-gene ribosome binding site (Shimatake et al, Nature (1981) 292:128). However, any available promoter system compatible with procaryotes can be used.

The expression systems useful in the eucaryotic systems of the invention comprise promoters derived from appropriate eucaryotic genes. A class of promoters useful in yeast, for example, include promoters for synthesis of glycolytic enzymes, including alcohol dehydrogenase-1 promoter, glyceraldehyde-3-phosphate dehydrogenase promoter (Holland & Holland, J Biol Chem (1980) 255:2596), alpha-factor promoter (Bitter et al, Proc Natl Acad Sci (1984) 81:5330), the gal promoter (Johnston & Davis, Mol Cell Biol (1984) 4:1440) those for 3-phosphoglycerate kinase (Hitzeman, et al, J Biol Chem (1980) 255:2073). Other promoters include those from the enolase gene (Hol-land, M.J., et al, J Biol Chem (1981) 256:1385) or the Leu2 gene obtained from YEp13 (Broach, J., et al, Gene (1978) 8:121).

Suitable mammalian promoters include the early and late promoters from SV40 (Fiers et al, Nature (1978) 273:113) or other viral promoters such as those derived from polyoma, adenovirus II, bovine papilloma virus or avian sarcoma viruses. Suitable viral and mammalian enhancers are cited above. In the event plant cells are used as an expression system, the nopaline synthesis promoter is appropriate (Depicker, A., et al, J Mol Appl Gen (1982) 1:561).

Depending on the host cell used, transformation is done using standard techniques appropriate to such cells. The calcium treatment employing calcium chloride, as described by Cohen, S.N., Proc Natl Acad Sci USA (1972) 69:2110, or the RbCl method described in Maniatis et al, Molecular Cloning: A Laboratory Manual (1982) Cold Spring Harbor Press, p. 254 is used for procaryotes or other cells which contain substantial cell wall barriers. Infection with Agrobacterium tumefaciens (Shaw, C.H., et al, Gene (1983) 23:315) is used for certain plant cells. For mammalian cells without such cell walls, the calcium phosphate precipitation method of Graham and van der Eb, Virology (1978) 52:546 is preferred. Transformations into yeast are carried out according to the method of Van Solingen, P., et al, J Bacter (1977) 130:946; and Hsiao, C.L., et al, Proc Natl Acad Sci USA (1979) 76:3829.

In general, after construction of a suitable expression system, the system is transfected into the appropriate host and successful transformants are selected by markers contained on the expression vectors. Successfully transformed colonies are then cultured in order to produce the desired protein. It is preferred that a promoter which can be controlled by regulating conditions in the environment be used so that the cells can be grown under conditions where the gene encoding the desired protein of the invention is not expressed, and then production of the protein induced by appropriate manipulation of conditions. For example, if the trp promoter is used in E. coli, the cells are grown in the presence of tryptophan and expression is then induced by diminution of tryptophan concentration or by addition of a tryptophan analog such as indolylacetic acid. If the gene is under control of the P_{L} promoter, the cells are grown at relatively low temperature, such as at about 35°C, to a suitable cell density, and the temperature is then elevated to activate this promoter. If produced in bacterial hosts as a mature intracellular protein, the N-terminal methionine may or may not be cleaved. In mammalian systems, for example, the use of the metallothionein promoter permits induction by addition of heavy metals or glucocorticoids. This protocol is preferred to prevent premature accumulation of the protein which may be harmful to the growth of the cell.

The protein may also be produced in secreted form by construction of vectors wherein the B peptide is preceded by a signal peptide workable in the appropriate host.

The protein is recovered from the medium or from the cells using suitable techniques generally known in the art, and purified by, for example, ion exchange chromatography, ammonium sulfate precipitation, gel permeation chromatography, and so forth. The sweetening compounds of the invention may also be used to generate antibodies immunospecific for themselves which are useful in purification of the compounds from synthetic or bioculture media. These antibodies may be prepared using standard immunization techniques by mixing the compound with suitable excipients and injecting the preparations into vertebrate hosts, typically rabbits or other mammalian hosts. The antisera or purified antibodies can then be conjugated to solid supports to provide immunoaffinity columns for purification of the desired materials.

The proteinaceous substances of the invention of the formula B-C-A, however prepared, can be used as sweetening elements in foods and beverages. Since the substances of the invention have a sweetening capacity at least 100 times that of sucrose, small quantities can be used to supplement the flavoring of juices, carbonated beverages, and other soft drinks. The sweeteners can also be used as sugar substitutes in hot beverages such as coffee and tea since the conformation is maintained at elevated temperatures. These materials can also be used to sweeten animal feeds.

For those embodiments of the invention compounds which are encoded by DNA sequences, the materials may be produced in situ by transfected microorganisms, eucaryotic cell lines, or plants. For example, expression systems containing the gene encoding the desired substance can be transfected into the culture organisms used in production of yogurt, wine, beer and the like and the sweetener produced along with the production of the desired product. In addition, expression systems operable in plants including the gene encoding the invention compounds may be used to transfect explants or plant protoplasts, and these then regenerated into intact plants which are then genetically capable of production of sweeter forms of fruit or vegetable products.

When used as a sweetener product, the compounds of the invention will be typically extended by addition of a liquid or powders wherein the compound of the invention constitutes about 0.1-99% by weight of the composition. Suitable extenders include, for example, inert powders such as cellulose and may include additional helpful ingredients such as antioxidants, preservatives, protease inhibitors, and so forth.

### Examples

The following examples are intended to illustrate the invention but not to limit its scope.

### Example 1

### Preparation of the Synthetic Gene

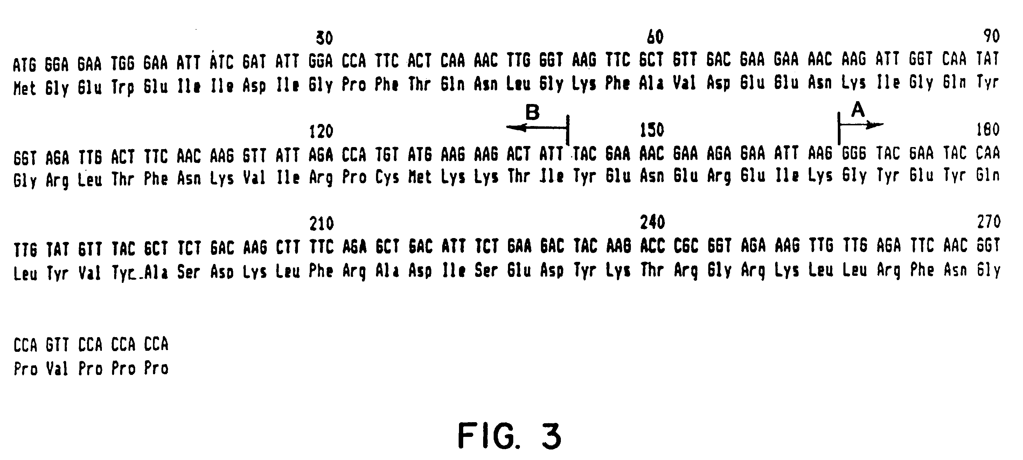

The protein of the amino acid sequence shown in Figure 3 is encoded by a DNA sequence as there shown. As shown in Figure 3, nucleotides 1-141 encode residues 1-45 of the native B chain preceded by a met encoding ATG start codon, nucleotides 142-165 encode the linking "C" portion of 8 amino acids, and nucleotides 166-285 encode residues 6-45 of the native A protein.

This synthetic gene was prepared from the following oligomers, synthesized using Applied Biosystems 380B DNA Synthesizer. 
5′ --> 3′
U1: TATGGGAGAATGGGAAATTATCGATATTGGACCATTCACTCAAAAC (46mer)
U2: TTGGGTAAGTTCGCTGTTGACGAAGAAAACAAGATTGGTCAATAT (45mer)
U3: GGTAGATTGACTTTCAACAAGGTTATTAGACCATGTATGAAGAAG (45mer)
U4: ACTATTTACGAAAACGAAAGAGAAATTAAGGGGTACGAATACCAA (45mer)
U5: TTGTATGTTTACGCTTCTGACAAGCTTTTCAGAGCTGACATTTCT (45mer)
U6: GAAGACTACAAGACCCGCGGTAGAAAGTTGTTGAGATTCAACGGT (45mer)
U7: CCAGTTCCACCACCATAATAG (21mer)
L1: CGATAATTTCCCATTCTCCCA (21mer)
L2: CGTCAACAGCGAACTTACCCAAGTTTTGAGTGAATGGTCCAATAT (45mer)
L3: CCTTGTTGAAAGTCAATCTACCATATTGACCAATCTTGTTTTCTT (45mer)
L4: CTCTTTCGTTTTCGTAAATAGTCTTCTTCATACATGGTCTAATAA (45mer)
L5: TGTCAGAAGCGTAAACATACAATTGGTATTCGTACCCCTTAATTT (45mer)
L6: TACCGCGGTCTTGTAGTCTTCAGAAATGTCAGCTCTGAAAAGCT (45mer)
L7: TCGACTATTATGGTGGTGGAACTGGACCGTTGAATCTCAACAACTTTC (48mer)
The oligomers were isolated by urea-polyacrylamide gel electrophoresis and purified by passing through a Sep-pak C18 column (Whatman) and annealed and ligated as shown: 
NdeI                                                    SalI
U1    U2    U3    U4    U5    U6    U7
L1    L2    L3    L4    L5    L6    L7
to obtain the synthetic gene of Figure 3 bracketed by NdeI and SalI sites.

For the ligation, each oligomer was phophorylated at 37°C for 45 min. in a reaction mixture of 30 ul containing 50 mM Tris-HCl, pH 8.0, 10 mM MgCl₂, 10 mM DTT, 1 mM ATP, and 5 units of T4 polynucleotide kinase. Each reaction mixture was pooled, extracted by phenol/chloroform, precipitated with ethanol, and dried under Speed-Vac. The dried pellet was dissolved in 50 ul distilled water and 7 ul ligation buffer (0.2 M Tris-HCl, pH 7.5, 0.1 M MgCl₂, 0.1 M DTT) added. The solution was placed in a 95°C water-bath and cooled slowly to room temperature overnight. To the mixture was added 7 ul of 10 mM ATP, 40 units of T4 DNA ligase (New England Biolab Inc.) and 2 ul of water.

The reaction mixture was kept at room temperature for 10 min., extracted by phenol/chloroform, precipitated, dried and redissolved in 85 ul water. The ligated oligomer mixture was treated with restriction endonuclease NdeI and SalI (New England Biolabs, Inc.), and the 290 base pair fragment was isolated by electrophoresis with a 7% polyacrylamide gel, the band electroeluted and purified using the Elutip-D column (S&S Co.).

M13mp19RF was used for cloning the synthetic monellin gene. M13mp19RF was cut with XbaI/SalI (New England Biolabs, Inc.), and the large fragment was isolated and purified. A synthetic XbaI/NdeI adaptor, 
XbaI NdeI
5′ - CTAGAAACTGCAATGTTGAATAAACGCTGATTTTCGATCA - 3′ (40mer)
3′ - TTTGACGTTACAACTTATTTGCGACTAAAAGCTAGTAT - 5′ (38mer)
was purified, and the NdeI/SalI digested, annealed synthetic monellin DNA fragment prepared above was combined with XbaI/SalI-treated M13mp19RF and XbaI/NdeI adaptor in 10 ul of 20 mM Tris-HCl, pH 7.5, 10 mM MgCl₂, 10 mM DTT, 200 units T4 DNA ligase (New England Biolabs, Inc.) and incubated at 4°C overnight to provide M13mp19 MON-1RF. The ligation mixture was transformed.into hosts by adding 5 ul of the ligation mixture to 200 ul of E. coli JM101 competent cells (Messing, J., Methods in Enzymology (1983) 101:20-78), and the desired sequence was confirmed by dideoxy sequencing (Sanger, T., et al, Proc Natl Acad Sci USA (1985) 74:5463-5467).

### Example 2

### Preparation of An Expression Vector

The 293 bp NdeI/SalI synthetic gene was isolated from M13mp19 MON-1 RF and purified. A commercially available vector, pDR720, containing the trp promoter/operator (Pharmacia, Inc.; Cat. #27-4930-01) was digested with SmaI/PvuII and blunt-end ligated to produce ptrp322. The ptrp322 was digested with HpaI/SalI and a 2.5 kb large fragment isolated. A synthetic HpaI/NdeI adaptor, 
5′ - AACTAGTACGCAAGTTCACGTAAAAAGGGTAATACA - 3′ (36mer)
3′ - TTGATCATGCGTTCAAGTGCATTTTTCCCATTATGTAT - 5′ (38mer)
HpaI NdeI
was synthesized using Applied Biosystems DNA Synthesizer Model 380B. The ligation reaction of the 293 bp synthetic monellin gene, HpaI/SalI-treated ptrp322 vector and the HpaI/NdeI synthetic adaptor was carried out in the presence of 10 ul of 20 mM Tris-HCl, pH 7.5, 10 mM MgCl₂, 10 mM DTT, and 200 units of T4 DNA ligase (N.E. Biolabs, Inc.) at 14°C overnight to give ptrp322H MON-1. The ligation mixture was transformed into E. coli W3110 (ATCC 27325) and ampicillin-resistant clones were picked.

### Example 3

### Production of B-C-A

An overnight culture of 50 ul of ptrp322H MON-1 in W3110 with Luria Broth was inoculated into 5 ml of M9 media containing 0.4% casamino acid, 10 ug/ml vitamin B1, 40 ug/ml ampicillin and cultured at 37°C in a temperature controlled-shaking incubator until OD₆₅₀ₙₘ reached about 0.5. Then 0.1 mg of indolyl acrylic acid was added to the reaction mixture to a concentration of 50 ug/ml and the mixture incubated further for about 8 hr. The cultured cells were pelleted at 2500 rpm for 5 min. in a Beckman J6 centrifuge. Laemmli protein sample buffer was added to the cell pellet, followed by heating at 95°C for 5 min. and the protein was loaded onto 15% Laemmli SDS - polyacrylamide gel (Laemmli, Nature (1970) 227:680-685). The electrophoresis was run at 300 for 2.5 hours. The gel was stained with Coomassie blue brilliant dye demonstrating a product having the correct molecular weight. The expressed product was isolated and shown to have a sweet taste.

The product was dissolved in water and heated to 100°C for 5 minutes. The solution was cooled to room temperature and the solution retasted. The sweet taste remained after the heating and cooling cycle.

### Example 4

### Purification of B-C-A

The cultured cells were sonicated in a buffer containing 0.01 M sodium phosphate, pH 7.2, 7 mM betamercaptoethanol, 1 mM EDTA, 25 ug/ml PMSF protease inhibitor. The sample was spun at 10,000 rpm for 15 min in a Sorvall SS34 rotor at 4°C. The extract was diluted with the buffer. The sample was loaded on CM-25 Sephadex column preequilibrated with 0.01 M phosphate buffer at pH 7.2. The column was washed with the same buffer for 3 hr. The product single chain monellin analog was eluted with 0.01 M phosphate buffer plus 0.1 M NaCl. After dialysis against water, the purity of the protein was determined by gel electrophoresis. If necessary, the protein was further purified by affinity column of Sepharose CI-4B charged with polyclonal monellin antibodies.

### Example 5

### Preparation of Alternate B-C-As

Following the above procedures, modified DNA sequences were prepared having different "C" linking sequences (between nucleotides 141 and 166 of Figure 3) as follows. 
Tyr-Glu-Asn-Arg-Glu-Asp-Ile-Lys
Tyr-Ala-Ser-Asp-Lys, and
Glu-Asp-Tyr-Lys-Thr-Arg-
The codons employed were the same as the codons indicated for the construct shown in Figure 3.

### Example 6

### Test for Sweetness

Sweetness of the product was assessed using an ordinary taste test. Comparison to the sweetness of sucrose was made by suitable dilutions on a weight basis. Compounds of the invention have at least the same sweetness as sucrose when diluted about 1000-fold in comparison.

In a typical test, 1, 10, 25 and 50 mg/ml aqueous sugar solution were used as standard solutions. The minimum weight of test compound which can be added to the water in order for it still to taste sweet to the tester is compared with the corresponding minimum weight of sucrose. The compounds of the invention require the addition of only an amount of material which is about 100-fold less than sucrose, for example, 60 ug/ml protein solution was as sweet as 50 mg/ml sugar (Lucky Supermarket's Lady Lee, brand sugar).

Stability under different pH conditions was measured by dissolving natural monellin (Worthington) and the product of Example 4 at 100 ug/ml concentration at pH 2, 4 and 6.3. Each sample was heated to 40, 50, 60, 70, 80, 90 and 100°C for 15 min, then let cool to room temperature before tasting. The most dramatic difference was that natural monellin lost its sweetness when heated to 50°C at pH 2 then let cool to room temperature for a taste test, while the product regained sweetness even after heating at 100°C for 5 min.

## Claims

1. A single chain proteinaceous compound having a sweetness property at least 50 times that of sucrose on a weight basis, of the formula B-C-A,
wherein B represents a peptide portion substantially equivalent to residues 1-46 of the B chain of native monellin;
C is a hydrophilic, physiologically acceptable covalent linker capable of providing a spacing length equivalent to a peptide of 3-10 amino acids; and
A represents a peptide substantially equivalent to residues 6-45 of the A chain of native monellin.

2. The compound of claim 1 wherein the B chain sequence is 
Met Gly Glu Trp Glu Ile Ile Asp Ile Gly
Pro Phe Thr Gln Asn Leu Gly Lys Phe Ala
Val Asp Glu Glu Asn Lys Ile Gly Gln Tyr
Gly Arg Leu Thr Phe Asn Lys Val Ile Arg
Pro Cys Met Lys Lys Thr Ile.
 or in which the initial met is absent.

3. The compound of claim 1 wherein the A chain sequence is

Gly Tyr Glu Tyr Gln
Leu Tyr Val Tyr Ala Ser Asp Lys Leu Phe
Arg Ala Asp Ile Ser Glu Asp Tyr Lys Thr
Arg Gly Arg Lys Leu Leu Arg Phe Asn Gly
Pro Val Pro Pro Pro

4. The compound of claim 1 wherein C is a hydrophilic oligomer containing 9-30 atoms in the linking segment.

5. The compound of claim 4 wherein C has the formula
A1-A2-A3-A4-A5-A6-A7-A8-A9-A10
wherein each of A1-A3 represents an amino acid residue and each of A4-A10 represents an amino acid residue or is not present, and wherein a majority of the amino acid residues are hydrophilic.

6. The compound of claim 5 wherein the amino acid residues are selected from those encoded by DNA.

7. The compound of claim 5 wherein A2, A4 and A6 are acidic amino acids; A5 and A8 are basic amino acids; A3 is a polar neutral amino acid; and A1 and A7 are nonpolar amino acids, and A9 and A10 are absent.

8. The compound of claim 7 wherein C has the formula Tyr-Glu-Asn-Glu-Arg-Glu-Ile-Lys.

9. The compound of claim 1 wherein the covalent linker conforms the B and A chains of monellin to substantially the native conformation.

10. The compound of claim 1 which is immunoreactive with antibodies raised against monellin.

11. The compound of claim 1 which is immunoreactive with antibodies raised against thaumatin.

12. A method to provide a sweet flavor to food compositions which comprises including a sweetening enhancing amount of the compound of claim 1 in said compositions.

13. A method to stabilize the three dimensional conformation of the B and A chains of monellin which comprises covalently binding a sequence substantially equivalent to residues 1-46 of the native B to a sequence substantially equivalent to residues 6-50 of the native A chain through a covalent linker which is hydrophilic, physiologically acceptable and equivalent in length to a 3-10 amino acid peptide.

14. The method of claim 13 wherein the covalent linker is a peptide.

15. A synthetic or recombinant DNA which encodes the compound of claim 6.

16. An expression system effective in expressing the DNA of claim 15 which comprises a first DNA encoding a compound of the formula B-C-A having a sweetness property at least 50 times that of sucrose on a weight basis,
wherein B represents a peptide portion substantially equivalent to residues 1-46 of the B chain of native monellin;
C is a peptide of 3-10 amino acids encoded by DNA, and,
A represents a peptide substantially equivalent to residues 6-45 of the A chain of native monellin,
operably linked to a second DNA sequence comprising a promoter functional in a compatible host.

17. A cell capable of producing a compound having a sweetness property at least 50 times that of sucrose on a weight basis, of the formula B-C-A,
wherein B represents a peptide portion substantially equivalent to residues 1-45 of the B chain of native monellin;
C is a peptide of 3-10 amino acids encoded by DNA; and
A represents a peptide substantially equivalent to residues 6-45 of the A chain of native monellin
which has been transformed with the expression system of claim 16.

18. A method to produce a compound having a sweetness property at least 50 times that of sucrose on a weight basis, of the formula B-C-A,
wherein B represents a peptide portion substantially equivalent to residues 1-46 of the B chain of native monellin;
C is a peptide of 3-10 amino acids encoded by DNA; and
A represents a peptide substantially equivalent to residues 6-45 of the A chain of native monellin, which comprises:
culturing the cells of claim 17,
exposing said cells to conditions favoring the functioning of the expression system, and
recovering the compounds from the culture.

19. A method to produce a compound having a sweetness property at least 50 times that of sucrose on a weight basis, of the formula B-C-A,
wherein B represents a peptide portion substantially equivalent to residues 1-46 of the B chain of native monellin;
C is a hydrophilic physiologically acceptable covalent linker capable of providing a spacing length equivalent to a peptide of 3-10 amino acids encoded by DNA; and
A represents a peptide substantially equivalent to residues 6-45 of the A chain of native monellin, which comprises:
a) preparing protected peptides bonded to solid supports wherein said one peptide has an amino acid sequence as recited for B and another peptide as recited for A,
b) removing the solid support from the peptides and deprotecting the peptides; and
c) ligating the peptides B and A to the covalent linker C to obtain B-C-A.

## Patentansprüche

1. Einkettige Proteinverbindung der Formel B-C-A mit süßen Eigenschaften, die mindestens dem 50-fachen der süßen Eigenschaften von Saccharose, bezogen auf das Gewicht, entsprechen,
worin B einen Peptidabschnitt darstellt, der weitgehend äquivalent zu den Resten 1-46 der B-Kette von nativem Monellin ist;
C ein hydrophiler, physiologisch verträglicher, kovalenter Linker ist, der in der Lage ist, eine Abstandslänge bereitzustellen, die einem Peptid mit 3 bis 10 Aminosäuren äquivalent ist; und
A ein Peptid darstellt, das weitgehend äquivalent zu den Resten 6-45 der A-Kette von nativem Monellin ist.

2. Verbindung nach Anspruch 1, worin die B-Kette folgende Sequenz aufweist: 
Met Gly Glu Trp Glu Ile Ile Asp Ile Gly
Pro Phe Thr Gln Asn Leu Gly Lys Phe Ala
Val Asp Glu Glu Asn Lys Ile Gly Gln Tyr
Gly Arg Leu Thr Phe Asn Lys Val Ile Arg
Pro Cys Met Lys Lys Thr Ile
 oder in der das Met am Anfang fehlt.

3. Verbindung nach Anspruch 1, worin die A-Kette folgende Sequenz aufweist:

Gly Tyr Glu Tyr Gln
Leu Tyr Val Tyr Ala Ser Asp Lys Leu Phe
Arg Ala Asp Ile Ser Glu Asp Tyr Lys Thr
Arg Gly Arg Lys Leu Leu Arg Phe Asn Gly
Pro Val Pro Pro Pro

4. Verbindung nach Anspruch 1, worin C ein hydrophiles Oligomeres ist, das 9-30 Atome im verknüpfenden Segment enthält.

5. Verbindung nach Anspruch 4, worin C die Formel
A1-A2-A3-A4-A5-A6-A7-A8-A9-A10
aufweist, worin jeder der Rest A1-A3 einen Aminosäurerest darstellt und jeder der Reste A4-A10 einen Aminosäurerest darstellt oder nicht vorhanden ist, und worin ein Großteil der Aminosäurereste hydrophil ist.

6. Verbindung nach Anspruch 5, worin die Aminosäurereste unter solchen Aminosäureresten ausgewählt sind, die von DNA codiert werden.

7. Verbindung nach Anspruch 5, worin A2, A4 und A6 saure Aminosäuren sind; A5 und A8 basische Aminosäuren sind; A3 eine polare neutrale Aminosäure ist; und A1 und A7 unpolare Aminosäuren sind und A9 und A10 fehlen.

8. Verbindung nach Anspruch 7, worin C die Formel Tyr-Glu-Asn-Glu-Arg-Glu-Ile-Lys aufweist.

9. Verbindung nach Anspruch 1, worin der kovalente Linker den B- und A-Ketten von Monellin im wesentlichen die native Konformation verleiht.

10. Verbindung nach Anspruch 1, die immunoreaktiv mit Antikörpern ist, die gegen Monellin erzeugt wurden.

11. Verbindung nach Anspruch 1, die immunoreaktiv mit Antikörpern ist, die gegen Thaumatin erzeugt wurden.

12. Verfahren, um Lebensmittelzusammensetzungen einen süßen Geschmack zu verleihen, daß das Einverleiben einer die Süße verstärkenden Menge der Verbindung nach Anspruch 1 in die Zusammensetzungen umfaßt.

13. Verfahren zur Stabilisierung der dreidimensionalen Konformation der B- und der A-Kette von Monellin, das das kovalente Binden einer Sequenz, die weitgehend äquivalent zu den Resten 1-46 der nativen B-Kette ist, an eine Sequenz, die weitgehend äquivalent zu den Resten 6-50 der nativen A-Kette ist, durch einen kovalenten Linker, der hydrophil, physiologisch verträglich und in seiner Länge einem Peptid mit 3-10 Aminosäuren äquivalent ist, umfaßt.

14. Verfahren nach Anspruch 13, wobei es sich bei dem kovalenten Linker um ein Peptid handelt.

15. Synthetische oder rekombinante DNA, die für die Verbindung nach Anspruch 6 codiert.

16. Zur Expression der DNA nach Anspruch 15 wirksames Expressionssystem, das eine erste DNA umfaßt, die für eine Verbindung der Formel B-C-A codiert, die süße Eigenschaften aufweist, die mindestens dem 50-fachen der süßen Eigenschaften von Saccharose, bezogen auf das Gewicht, entsprechen,
worin B einen Peptidanteil darstellt, der weitgehend äquivalent zu den Resten 1-46 der B-Kette von nativem Monellin ist;
C ein Peptid von 3-10 Aminosäuren ist, die durch DNA codiert werden, und
A ein Peptid darstellt, das weitgehend äquivalent zu den Resten 6-45 der A-Kette von nativem Monellin ist,
funktionell verknüpft an eine zweite DNA-Sequenz, die einen Promotor umfaßt, der in einem kompatiblen Wirt arbeitet, umfaßt.

17. Zur Herstellung einer Verbindung der Formel B-C-A mit süßen Eigenschaften, die mindestens dem 50-fachen der süßen Eigenschaften von Saccharose, bezogen auf das Gewicht, entsprechen, fähige Zelle,
worin B einen Peptidanteil darstellt, der weitgehend äquivalent zu den Resten 1-45 der B-Kette von nativem Monellin ist;
C ein Peptid von 3-10 Aminosäuren ist, die von DNA codiert werden; und
A ein Peptid darstellt, das weitgehend äquivalent zu den Resten 6-45 der A-Kette von nativem Monellin ist,
wobei die Zelle mit einem Expressionssystem nach Anspruch 16 transformiert worden ist.

18. Verfahren zur Herstellung einer Verbindung der Formel B-C-A mit süßen Eigenschaften, die mindestens dem 50-fachen der süßen Eigenschaften von Saccharose, bezogen auf das Gewicht, entsprechen,
worin B einen Peptidanteil darstellt, der weitgehend äquivalent zu den Resten 1-46 der B-Kette von nativem Monellin ist;
C ein Peptid mit 3-10 Aminosäuren ist, die von DNA codiert werden, und
A ein Peptid darstellt, das weitgehend äquivalent zu den Resten 6-45 der A-Kette von nativem Monellin ist, wobei das Verfahren folgende Stufen umfaßt:
Züchten von Zellen nach Anspruch 17,
Aussetzen der Zellen den Bedingungen, die eine Funktion des Expressionssystems begünstigen, und
Gewinnung der Verbindungen aus der Kultur.

19. Verfahren zur Herstellung einer Verbindung der Formel B-C-A mit süßen Eigenschaften, die mindestens dem 50-fachen der süßen Eigenschaften von Saccharose, bezogen auf das Gewicht, entsprechen,
worin B ein Peptidanteil darstellt, der weitgehend äquivalent zu den Resten 1-46 der B-Kette von nativem Monellin ist;
C ein hydrophiler physiologisch verträglicher kovalenter Linker ist, der in der Lage ist, eine Abstandslänge bereitzustellen, die äquivalent zu einem Peptid mit 3-10 Aminosäuren, die von DNA codiert werden, ist; und
A ein Peptid darstellt, das weitgehend äquivalent zu den Resten 6-45 der A-Kette von nativem Monellin ist, wobei das Verfahren folgende Stufen umfaßt:
a) Herstellung geschützter Peptide, die an feste Träger gebunden sind, wobei das eine Peptid eine Aminosäuresequenz aufweist, wie sie für B angegeben wurde, und das andere Peptid eine Aminosäuresequenz aufweist, wie sie für A angegeben wurde,
b) Entfernen des festen Trägers von den Peptiden und Entfernen der Schutzgruppen von den Peptiden; und
c) Verknüpfen der Peptide B und A mit dem kovalenten Linker C, um B-C-A zu erhalten.

## Revendications

1. Composé protéiné à chaîne unique, ayant une propriété édulcorante au moins égale à 50 fois, exprimée sur une base pondérale, celle du saccharose, de formule B-C-A, dans laquelle
B représente un fragment peptidique substantiellement équivalent aux résidus 1-46 de la chaîne B de la monelline native;
C est un segment de liaison covalente hydrophile, physiologiquement acceptable, capable de fournir un écartement de longueur équivalente à un peptide de 3 à 10 amino acides ; et
A représente un peptide substantiellement équivalent aux résidus 6-45 de la chaîne A de la monelline native.

2. Composé de la revendication 1 dans lequel la séquence de la chaîne B est 
Met Gly Glu Trp Glu Ile Ile Asp Ile Gly
Pro Phe Thr Gln Asn Leu Gly Lys Phe Ala
Val Asp Glu Glu Asn Lys Ile Gly Gln Tyr
Gly Arg Leu Thr Phe Asn Lys Val Ile Arg
Pro Cys Met Lys Lys Thr Ile.
 ou dans lequel le Met initial est absent.

3. Composé de la revendication 1 dans lequel la séquence de la chaîne A est

Gly Tyr Glu Tyr Gln
Leu Tyr Val Tyr Ala Ser Asp Lys Leu Phe
Arg Ala Asp Ile Ser Glu Asp Tyr Lys Thr
Arg Gly Arg Lys Leu Leu Arg Phe Asn Gly
Pro Val Pro Pro Pro

4. Composé de la revendication 1 dans lequel C est un oligomère hydrophile contenant 9 à 30 atomes dans le segment de liaison.

5. Composé de la revendication 4 dans lequel C a la formule
A1-A2-A3-A4-A5-A6-A7-A8-A9-A10
dans laquelle chacun des A1-A3 représente un résidu amino acide et chacun des A4-A10 représente un résidu amino acide ou n'est pas présent, et dans laquelle une majorité des résidus amino acide sont hydrophiles.

6. Composé de la revendication 5 dans lequel les résidus amino acide sont sélectionnés parmi ceux qui sont codés par l'ADN.

7. Composé de la revendication 5 dans lequel les A2, A4 et A6 sont des amino acides acides ; A5 et A8 sont des amino acides basiques ; A3 est un amino acide neutre polaire; et A1 et A7 sont des amino acides non polaires, et A9 et A10 sont absents.

8. Composé de la revendication 7 dans lequel C a la formule Tyr-Glu-Asn-Glu-Arg-Glu-Ile-Lys.

9. Composé de la revendication 1 dans lequel le segment de liaison covalente conforme les chaînes B et A de la monelline selon une conformation substantiellement identique à la native.

10. Composé de la revendication 1 qui est immunoréactif pour les anticorps fabriqués contre la monelline.

11. Composé de la revendication 1 qui est immunoréactif pour les anticorps fabriqués contre la thaumatine.

12. Méthode pour donner un goût sucré à des compositions alimentaires qui comprend l'inclusion d'une quantité de composé de la revendication 1 augmentant le goût sucré dans lesdites compositions.

13. Méthode pour stabiliser la conformation tridimensionnelle des chaînes B et A de la monelline qui comprend la liaison par covalence d'une séquence substantiellement équivalente aux résidus 1-46 de la chaîne B de la monelline native à une séquence substantiellement équivalente aux résidus 6-50 de la chaîne A de la monelline native par l'intermédiaire d'un segment de liaison covalente qui est hydrophile, physiologiquement acceptable et dont la longueur est équivalente à un peptide de 3 à 10 amino acides.

14. Méthode de la revendication 13 dans laquelle le segment de liaison covalente est un peptide.

15. ADN de synthèse ou recombinant qui code pour le composé de la revendication 6.

16. Système d'expression efficace pour exprimer l'ADN de la revendication 15 qui comprend un premier ADN codant pour pour un composé de formule B-C-A ayant une propriété édulcorante au moins égale à 50 fois, exprimée sur une base pondérale, celle du saccharose, de formule B-C-A, dans laquelle
B représente un fragment peptidique substantiellement équivalent aux résidus 1-46 de la chaîne B de la monelline native ;
C est un peptide de 3 à 10 amino acides codé par l'ADN et
A représente un peptide substantiellement équivalent aux résidus 6-45 de la chaîne A de la monelline native,
lié de façon opérable à une seconde séquence d'ADN comprenant un promoteur fonctionnel dans un hôte compatible.

17. Cellule capable de produire un composé ayant une propriété édulcorante au moins égale à 50 fois, exprimée sur une base pondérale, celle du saccharose, de formule B-C-A, dans laquelle
B représente un fragment peptidique substantiellement équivalent aux résidus 1-46 de la chaîne B de la monelline native ;
C est un peptide de 3 à 10 amino acides codé par l'ADN et
A représente un peptide substantiellement équivalent aux résidus 6-45 de la chaîne A de la monelline native,
qui a été transformée par le système d'expression de la revendication 16.

18. Méthode pour produire un composé ayant une propriété édulcorante au moins égale à 50 fois, exprimée sur une base pondérale, celle du saccharose, de formule B-C-A, dans laquelle
B représente un fragment peptidique substantiellement équivalent aux résidus 1-46 de la chaîne B de la monelline native ;
C est un peptide de 3 à 10 amino acides codé par l'ADN et
A représente un peptide substantiellement équivalent aux résidus 6-45 de la chaîne A de la monelline native, qui comprend :
la culture des cellules de la revendication 17,
l'exposition desdites cellules à des conditions favorisant le fonctionnement du système d'expression, et
la récupération des composés de la culture.

19. Méthode pour produire un composé ayant une propriété édulcorante au moins égale à 50 fois, exprimée sur une base pondérale, celle du saccharose, de formule B-C-A, dans laquelle
B représente un fragment peptidique substantiellement équivalent aux résidus 1-46 de la chaîne B de la monelline native;
C est un segment de liaison covalente hydrophile physiologiquement acceptable capable de donner un espacement d'une longueur équivalente à celle d'un peptide de 3 à 10 amino acides codé par l'ADN et
A représente un peptide substantiellement équivalent aux résidus 6-45 de la chaîne A de la monelline native, qui comprend :
a) la préparation des peptides protégés liés à des supports solides dans lesquels un desdits peptides a une séquence d'amino acides comme citée pour B, et l'autre desdits peptides comme cités pour A,
b) l'élimination du support solide des peptides et la déprotection des peptides ; et
c) la liaison des peptides B et A au segment de liaison covalente C pour donner B-C-A.
